# EUROPEAN PATENT SPECIFICATION

(11) **EP 0 343 671 B1**
(45) Date of publication and mention of the grant of the patent: **03.03.1993**
(21) Application number: 89109468.2
(22) Date of filing: 26.05.1989
(51) Int. Cl.: A61K 45/06, A61K 31/70

(54) **Pharmaceutical composition for the treatment of skin wounds**
Pharmazeutische Zusammensetzung für die Behandlung von Hautwunden
Composition pharmaceutique pour le traitement des blessures de la peau

(30) Priority: 27.05.1988 JP 131172/88
(43) Date of publication of application: 29.11.1989
(73) Proprietor: DAI-ICHI KOGYO SEIYAKU CO., LTD., Shimogyo-ku Kyoto (JP); Handa, Yasunobu, Sendai-shi Miyagi-ken (JP)
(72) Inventor: Handa, Yasunobu, Sendai-shi Miyagi-ken (JP); Nakamura, Shingo, Joyo-shi Kyoto-fu (JP)
(74) Representative: Kinzebach, Werner, Dr.

(56) References cited:
- WO-A-88/06880

## Description

The present invention relates to a pharmaceutical composition for the treatment of skin wounds such as pressure sore.

The external preparations heretofore used for the treatment of skin wounds generally comprise mixtures of antiseptics, antibacterial agents, antibiotics, antiinflammatory agents, proteolytic enzymes, exudate absorbents and hormones, though they vary in composition according to the etiology and severity of the wound to be treated. However, serious skin wounds such as high-degree burns, ambustions, pressure sores and traumas, which are far-reaching in extent and depth, involve intense inflammatory reactions, local circulatory disturbances and infections and are often refractory to treatment with the above-mentioned therapeutic compositions, treatment with antiseptics, or administration of antibiotics and antiinflammatory agents. At the affected sites, protein, water, minerals and vitamins are lost due to intense inflammatory reactions and the accompanying local circulatory disorder interferes with replenishment of these substances essential to tissue repair, thus setting off a vicious cycle. Therefore, infections are liable to develop at these sites and even if such infections are temporarily held in check by antisepsis or administration of antibiotics, delays in tissue repair tend to induce reinfections due to microbial substitution and emergence of resistant strains, thus forming extremely refractory sores or ulcers. The treatment modality adopted commonly, and as the best available one, these days in such skin wounds is to surgically cover up the exposed area of the wound with a patch of skin tissue, that is to say by skin grafting. However, even this procedure can be indicated only after the infection and inflammatory reaction have been sufficiently controlled and a drug therapy in the early stage of the wound is of paramount importance.

The use of fatty acids, fatty acid salts and sucrose esters in cosmetic compositions and other dermatological compositions is known. Various fatty acids, fatty acid salts and sucrose esters have also been employed in pharmaceutical compositions.

WO-A-88 06 880 discloses cosmetic base compositions adapted to topical application to tissue which comprise 0.1 to 15 % by weight of a sucrose fatty acid ester and 0.3 to 45 % by weight of an acyl fatty acid ester or alkali metal salt thereof and 50 to 99.6 % polar solvent. The compositions have utility as skin conditioners and cleansers and are capable of promoting wound healing, increasing total lipid synthesis, increasing thickness of epidermis layer, increasing cell proliferation, stimulating synthesis of glycosamino glycans and reducing skin dryness.

On the other hand, it has been found, and verified by the inventor of this invention as well, that sugars have a tissue repairing action and have actually been demonstrated clinically to produce a remarkable therapeutic effect on wounds (Japanese laid-open Patent Application No. 98260/1986). The present inventor has further found that potassium at high concentration also has a tissue repairing action, in addition to the activities to increase the viability of cells and tissues and promote their growth. Consequently, potassium has also been used as incorporated in sugar-containing therapeutic compositions for wounds. However, the use of these therapeutic agents has proved only moderately effective in cases accompanied by advanced circulatory disorder. In view of the importance of assuring an improvement in tissue regeneration disorder associated with local circulatory insufficiency as an adjunct to suppression of inflammatory reactions and prevention and treatment of infections in those burns, ambustions and pressure sores accompanied by advanced circulatory disorder, the inventor of this invention conducted a further research and ultimately discovered that various fatty acid esters of sugars have marked activities to improved local circulation and promote the regeneration and restoration of skin tissues.

The present invention has been developed on the basis of the above findings.

The present invention is accordingly directed to a pharmaceutical composition for the treatment of skin wounds which comprises at least one member selected from the group consisting of antiseptics, antibacterial agents and antibiotics (in this specification, this group of agents will be referred to sometimes as the anti-infective agent) and a sugar fatty acid ester, supplemented if necessary with at least one member selected from the group consisting of sugars, potassium chloride, antiinflammatory agents, proteolytic enzymes, exudate absorbents, vitamins, hormones and analogs and ointment bases.

As examples of the sugar moiety of said sugar fatty acid ester, there may be mentioned glucose, fractose, sucrose, lactose and sugar alcohols such as sorbitol. The fatty acid moiety of said sugar fatty acid ester is preferably derived from a saturated or unsaturated fatty acid containing 6 to 22 carbon atoms. As examples of the fatty acid moiety of said sugar fatty acid ester, there may be mentioned saturated or unsaturated intermediate and higher fatty acids such as caproic acid, caprylic acid, lauric acid, myristic acid, palmitic acid, stearic acid, behenic acid and oleic acid. Preferred, for practical purposes, are sucrose fatty acid esters which are commonly used in foods and drug products and available from commercial sources, i.e. esters of sucrose with stearic acid and palmitic acid.

The proportion of sugar fatty acid ester in the pharmaceutical composition of the invention is 2 to 40 grams to 100 grams of the total composition. The anti-infective agent used in the composition and its proportion may also be selected according to the intended use for the composition. By way of illustration, the following agents and amounts, based on 100 g of the composition, may be mentioned. Disinfectants: e.g. povidone iodine 5-10 ml, povidone iodine gel 20-60 g, sulfadiazine silver cream 10-25 g; antibacterial agents: e.g. ofloxacin 1-3 g; antibiotics: e.g. erythromycin 0.5-3 g, tetracycline hydrochloride 3g, fradiomycin sulfate 0.5 g, polymixin B sulfate 10⁶ U and gentamicin 0.1 g. In accordance with the present invention, the sugar fatty acid ester and anti-infective agent can be formulated with an ointment base such as Solbase^{R} and Macrogol^{R} 400. If necessary, sugars assisting in tissue regeneration, potassium chloride, antiinflammatory agents, hormones and analogs, proteolytic enzymes, exudate absorbents and vitamins may be incorporated in the composition. The following examples are intended to illustrate the effects of the pharmaceutical composition of this invention.

### Example 1

A 25-year-old male patient with paraplegia caused by an injury to the 8th thoracic cord (T₈). In January , continuous compression due to paralysis of lower extremities caused tissue necrosis in the form of a circle, 3 cm in diameter, beneath the skin of the left ankle with retention of exudates between the skin and the subcutaneous necrotic tissue. After debridement of the affected skin and necrotic tissue, an ointment comprising a mixture of 10 g of sucrose fatty acid ester (DK Ester F-50^{R}, Dai-ichi Kogyo Seiyaku Co., Ltd.) and 30 g of povidone iodine gel (Isodine^{R} Gel) was applied to the lesion twice daily. Twenty-four hours after the first application, which improved local circulation, the area of pressure sore became reddish and developed slight hemorrhage. A change suggestive of early granulation was found in the tissue surrounding the pressure sore. Exudates nearly disappeared in about 5 days and the ulcerative area became relatively dry. Granulation progressed rapidly and the skin tissue surrounding the pressure sore proliferated, resulting in gradual coverage of the affective area. Thus, complete healing was obtained about three weeks after initiation of treatment with the above-mentioned ointment.

### Example 2

A 36-year-old male patient with tetraplegia caused by an injury to the 7th cervical cord (C₇). Around March 5, 1988, a shallow oblong (major axis 5 cm x minor axis 3 cm) pressure sore was found in the center of the sacral region because of continuous compression. To this lesion, an ointment comprising a mixture of 20 g of sucrose fatty acid ester (DK Ester F-50^{R} Dai-ichi Kogyo Seiyaku Co., Ltd.), 30 g of Solbase^{R} 15 ml of Macrogol^{R} 400 and 2.5 g of Tarivid was applied twice daily. Twenty-four hours after the first application, the affected area became reddish and signs of granulation were found in the tissue surrounding the pressure sore. Thereafter, with the progression of granulation, the skin tissue proliferated from the surroundings and complete healing occurred in about two weeks after initiation of application.

### Example 3

The same patient as in Example 2. Around April 6, 1988, an oblong (major axis 8 cm x minor axis 5 cm) pressure sore was found in the left lumbar region, which was attributed to an abrasion sustained during an excercise. For this wound, an ointment comprising a mixture of 20 g of sucrose fatty acid ester (DK Ester F-50^{R}, Dai-ichi Kogyo Seiyaku Co., Ltd.), 30 g of Solbase^{R}, 15 ml of Macrogol^{R} 400 and 2.5 g of Tarivid was applied twice daily. As a result, following the same course as in Example 2, complete healing occurred in about 25 days.

### Example 4

A 54-year-old female patient. This patient had been confined to bed because of impaired consciousness due to subarachnoid hemorrhage (onset April 3, 1987). At the first visit (July 29, 1987), she had a deep round (13 cm in diameter) pressure sore, reaching the sacrum, in the center of the sacral region. Although Theradia was applied up to November 1987, no improvement occurred but rather a tendency of deterioration was noted. The central area of the pressure sore was necrotized and infected, producing a malodor and a large quantity of exudates. Therefore, from December 1 of the same year onward, an ointment comprising a mixture of 40 g of glucose and 60 g of povidone iodine gel (Isodine^{R} Gel) was applied three times daily. Consequently, the bacterial infection disappeared, the exudates gradually decreased in quantity and granulation occurred. However, probably because the patient did not move the body due to impaired consciousness and was in an extremely malnutritional status due to nasal feeding, the healing rate was slow. Then, the above ointment was replaced with an ointment comprising a mixture of 10 g of sucrose fatty acid ester (DK Ester F-50^{R}, Dai-ichi Kogyo Seiyaku Co., Ltd.), 30 g of glucose and 60 g of povidone iodine gel (Isodine^{R} Gel), which accerelated the healing rate and produced a sharp decrease in the amount of exudates and circulatory improvement in the ulcerative area. Granulation was activated and proliferation of the surrounding skin occurred. After six weeks, the diameter of the pressure sore had been reduced to about 10 cm. Subsequently, bacterial infection recurred and, therefore, the ointment was replaced with an ointment containing the antibacterial agent Tarivid^{R}. This ointment was composed of 7.5 g of sucrose fatty acid ester (DK Ester F-50, Dai-ichi Kogyo Seiyaku Co., Ltd.), 14 g of glucose, 40 g of Solbase^{R}, 20 ml of Macrogol^{R} 400 and 3 g of Tarivid^{R}. However, the subsequent healing rate was slow and even about two months later,no remarkable effect was found. So, the treatment regimen was changed to an ointment having sucrose fatty acid ester, glucose and vitamins as main ingredients. This ointment was composed of 30 g of sucrose fatty acid ester (DK Ester F-50^{R}, Dai-ichi Kogyo Seiyaku Co., Ltd.), 30 g of glucose, 60 g of glycerin, 20 g of purified water, 300 mg of vitamin E, 4 mg of vitamin A, 170 mg of vitamin B₆ and 7 g of Tarivid^{R}. As a result, exudates substantially ceased to come out and granulation and skin proliferation progressed rapidly so that the diameter of the pressure sore decreased to about 4 cm, indicating further healing.

These results suggested that the use of sucrose fatty acid ester in combination with glucose produces a synergistic effect on tissue repair and the addition of vitamins E, B₆ and A to this composition further increases the effect.

### Example 5

A 84-year-old male patient with right hemiplegia (onset December 12, 1984) due to cerebrovascular disorder. The patient had been confined to bed because of the above-mentioned disease and had an impaired nutritional status. At the first visit (November 27, 1985), a pressure sore, about 12 cm in diameter, associated with infection was found in the center of the sacral region. From December 1 of the same year onward, Isodine^{R} Glucose Gel Ointment comprising a mixture of 40 g of glucose and 60 g of povidone iodine gel (Isodine^{R} Gel) was applied three times daily. As a result, the bacterial infection disappeared and the pressure sore was reduced rapidly to the size of 8 cm (major axis) x 4 cm (minor axis) in March 1986. Around that time, there was a shift of the doctor in charge and the new doctor replaced the ointment so far used with Elase-C Ointment which was in prevalent use for pressure sore. However, the patient got better and, then, worse repeatedly, with no reduction of the pressure sore. Therefore, in May of the same year, Isodine^{R} Glucose Gel Ointment was reinstituted. During this treatment course, the above-mentioned ointment was temporarily replaced with Tarivid^{R} Glucose Gel Ointment (composition: 1.6 g of Tarivid^{R}, 5 g of glucose, 30 g of Solbase^{R}, 15 ml of Macrogol^{R} 400) because of a new bacterial infection. Granulation in the area of pressure sore and skin regeneration progressed gradually so that the pressure sore had been reduced to 4 cm (major axis) x 3 cm (minor axis) in October 1987. However, the pressure sore showed no further reduction. Therefore, on February 4, 1988, the ointment was replaced with an ointment comprising a mixture of 20 g of sucrose fatty acid ester (DK Ester F-160, Dai-ichi Kogyo Seiyaku Co., Ltd.), 30 g of Solbase^{R}, 15 ml of Macrogol^{R} 400 and 2.5 g of Tarivid^{R}. As a result , tissue repair resumed and the pressure sore healed nearly completely in early April 1988.

This case is worth attention because whereas inital treatments with an ointment containing an aseptic and glucose and an ointment containing a bactericide and glucose failed to cause a complete healing of the pressure sore despite marked improvement, an ointment containing an antibacterial agent and a sucrose fatty acid ester but containing no glucose produced a complete healing in a short time.

## Claims

1. A pharmaceutical composition for the external treatment of skin wounds comprising 2 to 40 percent of the total weight of the composition of a mono- or disaccharide fatty acid ester and a prophylactively effective amount of an anti-infective agent.

2. The pharmaceutical composition of Claim 1 wherein the mono- or disaccharide is glucose, fructose, sucrose, lactose or sorbitol.

3. The pharmaceutical composition of Claim 1 or 2 wherein the fatty acid is a saturated or unsaturated fatty acid containing 6 to 22 carbon atoms.

4. The pharmaceutical composition of anyone of the preceding Claims wherein the mono- or disaccharide fatty acid ester is a mono-, di, or triester of sucrose or a mixture thereof.

5. The pharmaceutical composition of anyone of the preceding Claims wherein said anti-infective agent is at least one member selected from antiseptics, antibacterial agents and antibiotics.

6. The pharmaceutical composition of anyone of the preceding Claims further comprising at least one member selected from mono- and disaccharides, potassium chloride, proteases, vitamins, hormones and ointment bases.

7. A process for preparing the compositions of claims 1 to 6 which comprises incorporating a mono- or disaccharide fatty acid ester and an anti-infective agent into a pharmaceutical carrier suitable for external administration.

## Patentansprüche

1. Pharmazeutische Zusammensetzung zur externen Behandlung von Hautwunden, enthaltend 2 bis 40 Prozent des Gesamtgewichts der Zusammensetzung eines Fettsäuremono- oder -disaccharidesters und eine prophylaktisch wirksame Menge eines antiinfektiösen Mittels.

2. Pharmazeutische Zusammensetzung nach Anspruch 1, worin das Mono- oder Disaccharid Glukose, Fructose, Saccharose, Laktose oder Sorbit ist.

3. Pharmazeutische Zusammensetzung nach Anspruch 1 oder 2, worin die Fettsäure eine gesättigte oder ungesättigte Fettsäure mit 6 bis 22 Kohlenstoffatomen ist.

4. Pharmazeutische Zusammensetzung nach einem der vorhergehenden Ansprüche, worin der Fettsäuremono- oder -disacharidester ein Mono-, Di- oder Triester von Saccharose oder eine Mischung davon ist.

5. Pharmazeutische Zusammensetzung nach einem der vorhergehenden Ansprüche, worin das antiinfektiöse Mittel zumindest ein Mittel ist, das unter Antiseptika, antibakteriellen Mitteln und Antibiotika ausgewählt ist.

6. Pharmazeutische Zusammensetzung nach einem der vorhergehenden Ansprüche, die zusätzlich zumindest einen Bestandteil enthält, der unter Mono- und Disachariden, Kaliumchlorid, Proteasen, Vitaminen, Hormonen und Salbengrundlagen ausgewählt ist.

7. Verfahren zur Herstellung der Zusammensetzungen der Ansprüche 1 bis 6, wobei man einen Fettsäuremono- oder -disaccharidester und ein antiinfektiöses Mittel einem zur externen Verabreichung geeigneten pharmazeutischen Träger einverleibt.

## Revendications

1. Composition pharmaceutique pour le traitement externe des plaies cutanées, comprenant 2 à 40 % du poids total de la composition d'un ester d'acide gras de mono- ou de disaccharide et une quantité efficace du point de vue prophylactique d'un agent anti-infectieux.

2. Composition pharmaceutique selon la revendication 1, dans laquelle le mono- ou disaccharide est le glucose, le fructose, le saccharose, le lactose ou le sorbitol.

3. Composition pharmaceutique selon les revendications 1 ou 2, dans laquelle l'acide gras est un acide gras saturé ou insaturé en C₆ à C₂₂.

4. Composition pharmaceutique selon l'une quelconque des revendications précédentes, dans laquelle l'ester d'acide gras de mono- ou disaccharide est un mono-, di- ou tri-ester de saccharose ou un mélange de ceux-ci.

5. Composition pharmaceutique selon l'une quelconque des revendications précédentes, dans laquelle cet agent anti-infectieux est au moins un membre choisi parmi les antiseptiques, les agents antibactériens et les antibiotiques.

6. Composition pharmaceutique selon l'une quelconque des revendications précédentes, comprenant en outre au moins un membre choisi parmi les mono- et disaccharides, le chlorure de potassium, les protéases, les vitamines, les hormones et des bases pour pommades.

7. Procédé pour la préparation des compositions selon les revendications 1 à 6, qui comprend l'incorporation d'un ester d'acide gras de mono- ou disaccharide et d'un agent anti-infectieux à un support pharmaceutique convenant pour l'administration externe.
